(19) **Europäisches Patentamt**

**European Patent Office**

**Office européen des brevets**

(11) **EP 1 460 378 A2**

(12) **EUROPÄISCHE PATENTANMELDUNG**

(43) Veröffentlichungstag:
22.09.2004 Patentblatt 2004/39

(51) Int Cl.7: **G01C 17/38**, G01C 21/16

(21) Anmeldenummer: 04002975.3

(22) Anmeldetag: **11.02.2004**

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(30) Priorität: **17.03.2003 DE 10312154**

(71) Anmelder: **Fraunhofer-Gesellschaft zur Förderung der angewandten Forschung e.V. 80636 München (DE)**

(72) Erfinder:
- **Haid, Markus, Dipl.-Ing.**
  **70567 Stuttgart (DE)**
- **Marquardt, Gerhard, Dipl.-Ing.**
  **72074 Tübingen (DE)**
- **Breitenbach, Jan, Dr.**
  **70563 Stuttgart (DE)**

(74) Vertreter: **Dreiss, Fuhlendorf, Steimle & Becker Patentanwälte Postfach 10 37 62 70032 Stuttgart (DE)**

(54) **Verfahren und Vorrichtung zum Ausführen einer Objektverfolgung**

(57) Die Erfindung betrifft ein Verfahren zum Ausführen einer Objektverfolgung, wobei mittels einer drei Drehratensensoren umfassenden Vorrichtung Messdaten ermittelt und hieraus durch Integration drei Drehwinkel zur weiteren Bestimmung der Orientierung des Objekts im Raum errechnet werden, wobei die Ermittlung der Messdaten und die Errechnung der drei Drehwinkel quasikontinuierlich entsprechend einer Abtastrate durchgeführt wird; um zumindest während einer beschränkten Zeitdauer eine hohe Genauigkeit zu erreichen, ist das Verfahren dadurch gekennzeichnet, dass vor Beginn der Objektverfolgung und dann immer wieder sobald ein Ruhen der Vorrichtung für eine bestimmte Zeitdauer festgestellt wird ein Offset-Wert des Ausgangssignals der Drehratensensoren ermittelt wird und nachfolgend bis zur nächstfolgenden Ermittlung dieser Offset-Wert für die jeweiligen Drehratensensoren in Abzug gebracht wird, so dass er nicht in die Integration eingeht, und dass eine Abweichung der Orientierung der Achsen der drei Drehratensensoren der Vorrichtung von einer angenommenen Orientierung der Achsen zueinander und zu der Vorrichtung und der hieraus bei der Berechnung der Drehwinkel aus den Drehratensensorsignalen resultierende Fehler durch Anwendung einer diese Abweichung berücksichtigenden und vor Beginn der Objektverfolgung ermittelten Ausgleichsmatrix kompensiert wird.

Fig 3

EP 1 460 378 A2

Printed by Jouve, 75001 PARIS (FR)

**Beschreibung**

Die Erfindung betrifft ein Verfahren und eine Vorrichtung zum Ausführen einer Objektverfolgung, wobei mittels einer drei Drehratensensoren umfassenden Vorrichtung Messdaten ermittelt und hieraus durch Integration drei Drehwinkel zur weiteren Bestimmung der Orientierung des Objekts im Raum errechnet werden, wobei die Ermittlung der Messdaten und die Errechnung der drei Drehwinkel quasikontinuierlich entsprechend einer Abtastrate durchgeführt wird und wobei die Orientierung des Objekts im Raum angezeigt oder in an sich beliebiger Weise weiterer Datenverarbeitung zugeführt oder zu Steuerungsprozessen verwendet werden kann.

Ein Verfahren und eine Vorrichtung der vorstehend genannten Art ist bekannt aus US 5,645,077. Gemäß dieser Druckschrift wird die Orientierung einer auf den Kopf eines Benutzers aufsetzbaren Sensorvorrichtung und damit die Orientierung des Kopfs des Benutzers im Raum (dreidimensional) erfasst. Hierfür werden drei Drehratensensoren, die an der aufsetzbaren Vorrichtung vorgesehen sind, benutzt. Diese drei Drehratensensoren liefern Messdaten, aus denen durch Integration Drehwinkel um die jeweiligen Achsen der Drehratensensoren errechenbar sind. Die Messdaten sowie die hieraus errechneten Drehwinkel sind fehler- bzw. driftbehaftet. Schließlich ist die aus den Drehwinkeln ermittelte Orientierung fehlerhaft. Um diesem Fehler bei der Ermittlung der Orientierung entgegenzuwirken, wird zwar zu Beginn einmalig ein Offset der Drehratensensoren ermittelt und für eine Korrektur berücksichtigt. Eine den Anforderungen genügende Kompensation wird gemäß dieser Druckschrift aber erreicht, indem durch von den drei Drehratensensoren unterschiedliche Mittel, nämlich durch einen Gravitationskraftneigungssensor oder durch Magnetfeldsensoren oder beide, die wirkliche Orientierung im Raum periodisch, etwa alle 10 Sekunden, gemessen wird, und zwar dann, wenn sich der Kopf für eine gewisse Zeit in Ruhe befindet. In einem Fehlerkompensator wird dann der Unterschied zwischen dieser mit hoher Genauigkeit ermittelten Orientierung zu der aus dem Signal der drei Drehratensensoren ermittelten Orientierung durch ein "resetting" beseitigt. - Bei diesem Verfahren gemäß US 5,645,077 ist es daher zwingend erforderlich, dass auch bei der Messung über verhältnismäßig kurze Zeitspannen zwingend über von den drei Drehratensensoren unterschiedliche Mittel die tatsächliche Orientierung periodisch gemessen und zugrundegelegt wird. Dies kann in einem einzigen Korrektur- bzw. Reset-Vorgang oder durch einen Satz von Korrektursignalen erreicht werden, die dann aufeinanderfolgend angewandt werden. Hierbei kann auch ein Kalman-Filter Verwendung finden, der statistische Daten über die Kopfbewegung bei Menschen verwenden kann.

US 5,953,683 lehrt in Abgrenzung zu US 5,645,077 die Verwendung von nur einem oder höchstens zwei Drehratensensoren. Hierfür ist es jedoch zwingend erforderlich, dass weitere Sensoren bei der Ermittlung der Orientierung mitwirken: Nach einer ersten Ausführungsform von US 5,953,683 wird ein dreiachsiger Magnetfeldsensor zusammen mit genau einem Drehratensensor verwendet. Über den Drehratensensor wird die Steigung (elevation) oder die Neigung (roll) gemessen, während unter Verwendung des dreiachsigen Magnetfeldsensors der Azimut-Winkel und die Neigung oder die Steigung bestimmt werden.

Gemäß einem zweiten Ausführungsbeispiel wird ein dreiachsiger Beschleunigungssensor und ein zweiachsiger Drehratensensor, also zwei bezüglich zweier Richtungen ausgerichtete und sensitive Drehratensensoren, verwendet. Die Verwendung von Beschleunigungssensoren ist jedoch nur so lange einfach handhabbar, so lange keine translatorische Bewegung hinzukommt. Schließlich wird nach einer dritten Ausführungsform ein dreiachsiger Beschleunigungssensor und ein einziger Drehratensensor verwendet. Auch gemäß dieser Druckschrift wird während eines temporären Stillstands zur Verbesserung des Driftproblems der Integrator "resettet" oder zurückgesetzt. Dennoch ist für die Berechnung der drei Orientierungswinkel für die Steigung, die Neigung und den Azimut das Zusammenwirken der vorstehend genannten Sensoren zwingend erforderlich. Gemäß dieser Druckschrift soll ausdrücklich von der Verwendung dreier Drehratensensoren Abstand genommen werden.

Der vorliegenden Erfindung liegt die Aufgabe zugrunde, ein Verfahren der eingangs genannten Art dahingehend zu verbessern, dass die Ermittlung der Orientierung eines Objekts im Raum nur unter Verwendung der drei Drehratensensoren durchführbar ist und zumindest für eine beschrankte Zeitdauer den in der Praxis bei vielen Anwendungen gestellten Genauigkeitserfordernissen genügt.

Diese Aufgabe wird bei einem Verfahren der genannten Art erfindungsgemäß dadurch gelöst, dass vor Beginn der Objektverfolgung und dann immer wieder sobald ein Ruhen der Vorrichtung für eine bestimmte Zeitdauer festgestellt wird ein Offset-Wert des Ausgangssignals der Drehratensensoren ermittelt wird und nachfolgend bis zur nächstfolgenden Ermittlung dieser Offset-Wert für die jeweiligen Drehratensensoren in Abzug gebracht wird, so dass er nicht in die Integration eingeht, und dass eine Abweichung der Orientierung der Achsen der drei Drehratensensoren der Vorrichtung von einer angenommenen Orientierung der Achsen zueinander und zu der Vorrichtung und der hieraus bei der Berechnung der Drehwinkel aus den Drehratensensorsignalen resultierende Fehler durch Anwendung einer diese Abweichung berücksichtigenden und vor Beginn der Objektverfolgung ermittelten Ausgleichsmatrix kompensiert wird.

Es wurde festgestellt, dass die konstruktionsbedingt nicht zu vermeidende Abweichung der Orientierung der Achsen der drei Drehratensensoren von einer angenommenen Orientierung bei der Berechnung der Drehwinkel sehr rasch zu Ungenauigkeiten fuhrt. Dadurch, dass dieser Fehler durch Anwendung einer für das betreffende System zu bestimmenden Ausgleichsmatrix kompensiert wird, lässt sich für praktische Anwendungen eine Steigerung der Genauigkeit erreichen.

Des weiteren erweist es sich als vorteilhaft, dass ein stets vorhandener Offset-Wert immer wieder in einer Ruhelage der Vorrichtung ermittelt wird und bei jeder Berechnung der neuen Orientierung des Objekts, also bei jeder Abtastung oder Messwertnahme durch die Drehratensensoren in Abzug gebracht wird.

Zur Ermittlung der Ausgleichsmatrix zur Kompensation der Abweichung der Orientierung der Achsen wird vor Beginn der Objektverfolgung eine rotierende Bewegung um eine jeweilige Achse der die Drehratensensoren tragenden Vorrichtung unter Stillsetzung der beiden anderen Achsen durchgeführt und anhand der hierbei erhaltenen Drehratensensorsignale die Ausgleichsmatrix errechnet.

Durch Anwendung des erfindungsgemäßen Verfahrens wird erreicht, dass eine Orientierung eines Objekts im Raum bei Verwendung von nur drei Drehratensensoren, ohne dass weitere Sensoren zur Korrektur der Ergebnisse verwendet werden müssen, über eine verhältnismäßig lange Zeit mit einem sehr geringen Fehler erhalten werden kann. Unter Anwendung des erfindungsgemäßen Verfahrens ist es möglich, dass die Genauigkeit besser als 4° Abweichung während einer Zeitdauer von 5 min ist. Dies ermöglicht es, für ein großes Anwendungsgebiet von Prozessen eine Objektverfolgung unter Bestimmung der Orientierung des Objekts durchzuführen, und zwar nur unter Verwendung dreier Drehratensensoren, sofern der zu beurteilende Zeitraum 5 min nicht wesentlich überschreitet. Beispielsweise kommen hierfür Anwendungen in Frage wie z. B. eine Scanprozess, bei dem ein handgeführter oder während des Scanvorgangs verschwenkbarer Messkopf unter Durchführung des erfindungsgemäßen Verfahrens verfolgt werden kann. Auf diese Weise können die im Zuge des Scannens erfassten Daten entsprechend der ermittelten Neigung des Messkopfs, die ausschließlich unter Verwendung der drei Drehratensensoren ermittelt werden kann, ausgewertet werden. Dies stellt jedoch nur ein Beispiel für einen üblicherweise verhältnismäßig kurzzeitigen Prozess dar, bei dem die Orientierung eines Objekts im Raum eine Rolle spielen kann.

Die Genauigkeit der Bestimmung der Orientierung wird nach einer Ausführungsform der Erfindung auch dadurch erhöht, dass bei jeder Messdatennahme und Ermittlung der drei Drehwinkel ein Quaternionen-Algorithmus der nachfolgend definierten Art auf die drei Drehwinkel angewandt wird, um hieraus die aktuelle Orientierung des Objekts im Raum zu errechnen. Die hierdurch erzielte weitere Verbesserung beruht auf folgendem Umstand: Wenn man die durch einfache Integration bei jedem infinitesimalen Schritt der Abtastung, d. h. der Messdatennahme erhaltbaren infinitesimalen Drehwinkel um eine jeweilige Achse zur Bestimmung der Orientierungsänderung des Objekts so heranziehen würde, dass die Drehungen nacheinander um die jeweilige Achse durchgeführt würden, so würde hieraus ein Fehler resultieren. Dieser Fehler liegt darin begründet, dass die Messdaten der drei Sensoren zum gleichen Zeitpunkt genommen werden, weil eine Drehung in der Regel gleichzeitig um drei Achsen stattfindet und ermittelt wird. Wenn dann zur Ermittlung der Positionsveränderung die drei bestimmten Drehwinkel aber nacheinander als Drehungen um die jeweiligen Achsen berücksichtigt würden, so würde bei der Drehung um die zweite und die dritte Achse ein Fehler resultieren, da diese Achsen bereits im Zuge der ersten Drehung fehlerbehaftet in eine andere Orientierung gebracht wurden. Dem wird durch Anwendung des Quaternionen-Algorithmus auf die drei Drehwinkel begegnet. Auf diese Weise werden die drei Drehungen durch eine einzige Transformation ersetzt. Der Quaternionen-Algorithmus ist folgendermaßen definiert:

Die Quaternion ist in Gl.1. definiert:

$$(1) \qquad \underline{q} = q_0 + q_1 \cdot i + q_2 \cdot j + q_3 \cdot k$$

mit den Bedingungen Gl.2. bis Gl.6.

$$(2) \qquad\qquad q_{0..3} \in \Re$$

$$(3) \qquad i^2 = j^2 = k^2 = 1$$

$$(4) \qquad i \cdot j = -j \cdot i = k$$

$$(5) \qquad j \cdot k = -k \cdot j = i$$

$$(6) \qquad k \cdot i = -i \cdot k = j$$

Durch Zusammenfassen der komplexen Anteile zu einem Vektor v und mit $q_0$=w gelangt man zu der Schreibweise in Gl.7.

$$(7) \qquad \underline{q} \in (w{\cdot}\underline{v})^{\mathsf{T}}$$

mit den Bedingungen Gl.2. bis Gl.6.

$$(8) \qquad\qquad\qquad w \in \mathfrak{R}$$

$$(9) \qquad\qquad\qquad \underline{v} \in \mathfrak{R}^3$$

Für die Benutzung der Quaternionen gelten die Definitionen Gl. 10 bis Gl.15.
Konjugierte Quaternion

$$(10) \qquad\qquad \underline{q}^k = \begin{pmatrix} w \\ -\underline{v} \end{pmatrix}$$

Betrag

$$(11) \qquad\qquad \left| \underline{q} \right| = \sqrt{w^2 + \underline{v} \cdot \underline{v}}$$

Inversion

$$(12) \qquad\qquad \underline{q}^{-1} = \frac{\underline{q}^k}{\left| \underline{q} \right|}$$

Multiplikation

$$(13) \qquad \underline{q}_1 \cdot \underline{q}_2 = \begin{pmatrix} w_1 \\ \underline{v}_1 \end{pmatrix} \cdot \begin{pmatrix} w_2 \\ \underline{v}_2 \end{pmatrix} = \begin{pmatrix} w_1 \cdot w_2 + \underline{v}_1 \cdot \underline{v}_2 \\ w_1 \cdot \underline{v}_2 + w_2 \cdot \underline{v}_1 + \underline{v}_1 \times \underline{v}_2 \end{pmatrix}$$

Darstellung eines Vektors

$$(14) \qquad\qquad \underline{q}_v = \begin{pmatrix} 0 \\ \underline{v} \end{pmatrix}$$

Darstellung eines Skalars

$$(15) \qquad \underline{q}_w = \begin{pmatrix} w \\ \underline{0} \end{pmatrix}$$

Besondere Bedeutung für die inertiale Objektverfolgung erhält die Multiplikation. Diese stellt eine Rotation einer Quaternion dar. Dafür wird eine Rotationsquaternion Gl.16. eingeführt.

$$(16) \qquad \underline{q}_{rot} = \begin{pmatrix} \cos\left(\frac{|\underline{\phi}|}{2}\right) \\ \sin\left(\frac{|\underline{\phi}|}{2}\right) \cdot \frac{\underline{\phi}}{|\underline{\phi}|} \end{pmatrix}$$

Der Vektor $\phi$ besteht aus den einzelnen Drehungen um die Koordinatenachsen.

Eine Drehung eines Punkts oder Vektors kann nun wie folgt errechnet werden. Zunächst müssen die Koordinaten des Punkts oder der Vektor mittels Gleichung Gl.14 in eine Quaternion umgewandelt und anschließend die Multiplikation mit der Rotationsquaternion durchgeführt werden (Gl.16.). Die Ergebnisquaternion enthält den rotierten Vektor in der gleichen Schreibweise. Unter der Voraussetzung, dass der Betrag einer Quaternion gleich eins ist, kann die invertierte Quaternion durch die konjugierte ersetzt werden (Gl.17).

$$(17) \qquad \underline{q}_v = \underline{q}_{rot} \cdot \underline{q}_v \cdot (\underline{q}_{rot})^{-1} = \underline{q}_{rot} \cdot \underline{q}_v \cdot (\underline{q}_{rot})^{-k}$$

wegen Gl.18.

$$(18) \qquad |\underline{q}_{rot}| = 1$$

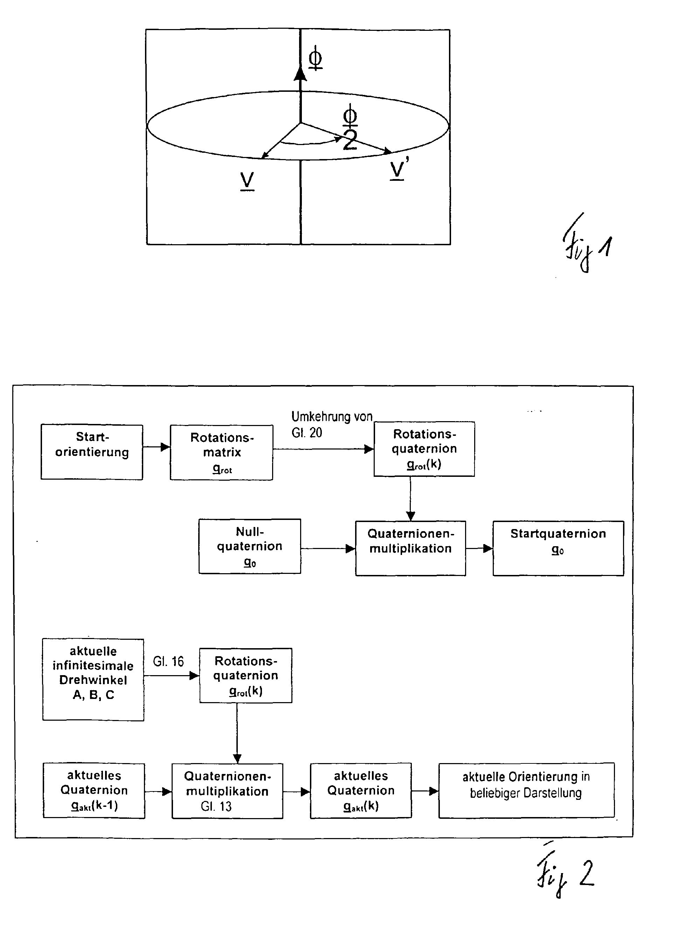

Wie kann man diese Operation verdeutlichen? Der Vektor $\phi$ ist der Normalenvektor zu der Ebene, in der eine Drehung um den Winkel $\frac{1}{2}\phi$ ausgeführt wird. Der Winkel entspricht dem Betrag des Vektors $\phi$. Siehe Fig.1.

Aus Fig.1. ist ersichtlich, dass eine Rotation in jeder beliebigen Ebene und unter Angabe nur eines Winkels durchgeführt werden kann. Darin zeigen sich auch die besonderen Vorteile für dieses Verfahren. Weitere Vorteile sind die geringe Anzahl an notwendigen Parametern und trigonometrischer Funktionen, welche durch Näherungen für kleine Winkel völlig ersetzt werden konnten. Fur die Rotation mit dem Vektor $\omega$ gilt die Differenzialgleichung:

$$(19) \qquad \frac{d}{dt}\underline{q}_{rot} = \frac{1}{2}\underline{q}_{rot} \cdot \begin{pmatrix} 0 \\ \omega \end{pmatrix}$$

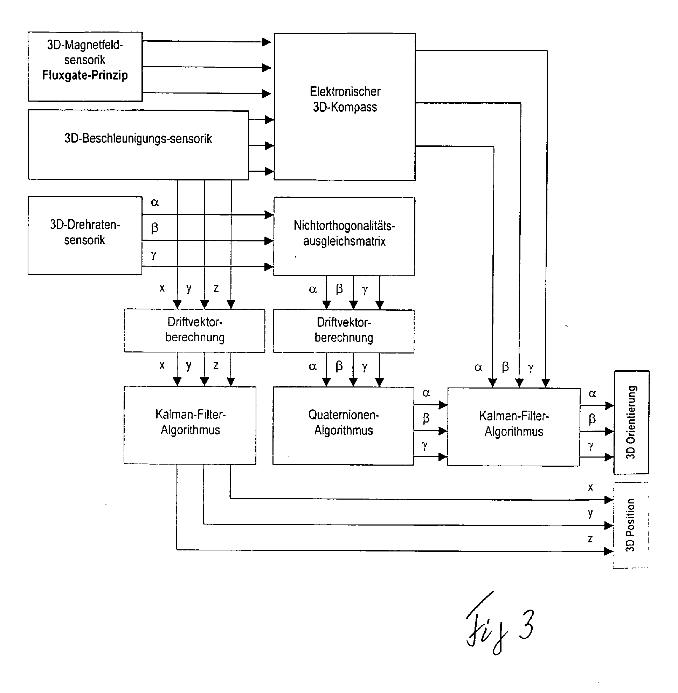

Die konkrete Umsetzung des Quaternionen Algorithmus ist in Figur 2 dargestellt und erfolgt folgendermaßen: Insgesamt erfolgt die gesamte Berechnung mit Hilfe von Einheitsvektoren. Ausgehend von der Startorientierung werden die Starteinheitsvektoren $E_x$, $E_y$ und $E_z$ ermittelt.

Aus den Einheitsvektoren wird mit Hilfe von Gleichung Gl. 20 die Rotationsmatrix berechnet.

$$(20) \quad R = \begin{bmatrix} (q_0)^2 + (q_1)^2 - (q_2)^2 - (q_3)^2 & 2 \cdot (q_1 \cdot q_2 - q_0 \cdot q_3) & 2 \cdot (q_1 \cdot q_3 - q_0 \cdot q_2) \\ 2 \cdot (q_1 \cdot q_2 - q_0 \cdot q_3) & (q_0)^2 - (q_1)^2 + (q_2)^2 - (q_3)^2 & 2 \cdot (q_2 \cdot q_3 - q_0 \cdot q_1) \\ 2 \cdot (q_1 \cdot q_3 - q_0 \cdot q_2) & 2 \cdot (q_2 \cdot q_3 - q_0 \cdot q_1) & (q_0)^2 - (q_1)^2 - (q_2)^2 + (q_3)^2 \end{bmatrix}$$

Ausgehend von einer Startorientierung des mit dem Objekt verbundenen Koordinatensystems, insbesondere ausgehend von sogenannten Einheitsstartvektoren, wird gemäß Gleichung 20 die Rotationsmatrix R, bei der es sich um eine 3x3-Matrix handelt, errechnet. Aus einer Umkehrung dieser Gleichung 20 lässt sich ein Rotationsquaternion $q_{rot}$ (k) erhalten. Mit Hilfe des Nullquaternions, welches sich aus den Nulleinheitsvektoren ergibt, wird durch eine Multiplikation mit dem Rotationsquaternion das Startquarternion berechnet. Bei der nächstfolgenden Abtastung, also bei der nächsten Messdatennahme und Integration zu aktuellen infinitesimalen Drehwinkeln A, B, C errechnet sich dann ein für diesen Schritt zu benutzendes Rotationsquaternion $q_{rot}$ (k). Das im vorherigen Schritt gebildete Quaternion $q_{akt}$ (k-1) wird dann mit diesem Rotationsquaternion $q_{rot}$ (k) gemäß Gleichung 13 multipliziert, um das aktuelle Quaternion des vorliegenden k-ten Schritts, nämlich $q_{akt}$ (k) zu erhalten. Aus diesem aktuellen Quaternion kann dann für den gerade durchgeführten Abtastschritt die aktuelle Orientierung des Objekts in beliebiger Darstellung angegeben werden.

Wenn vorausgehend davon die Rede ist, dass bei Ausführung des erfindungsgemäßen Verfahrens unter Verwendung von nur drei Drehratensensoren eine hohe Genauigkeit bei der Ermittlung der Orientierung des Objekts im Raum erreicht werden kann, und zwar über eine verhältnismäßig lange Zeitspanne im Bereich von einigen Minuten, insbesondere im Bereich von wenigstens 3, vorzugsweise von wenigstens 4 und in besonders bevorzugter Weise von wenigstens 5 min eine Abweichung von weniger als 4° erreicht werden kann, so bedeutet dies nicht, dass keine zusätzlichen Sensoren bei der Objektverfolgung zum Einsatz kommen dürfen. So erweist es sich, insbesondere bei Objektverfolgungsprozessen, die über einen Zeitraum von mehr als 5 min andauern, als vorteilhaft, wenn zusätzlich mittels Inertialsensoren die Erdbeschleunigung gemessen wird. Da die Erdbeschleunigung stets gleich gerichtet ist und an einem bestimmten Ort einen bestimmten Betrag aufweist, ist es möglich, hieraus in Kombination mit Magnetfeldsensoren (elektronischer Kompass) weitere Informationen zur Ermittlung der Orientierung des Objekts im Raum zu erhalten. Beispielsweise können hieraus auch translatorische Bewegungen errechnet werden.

Es erweist sich als vorteilhaft, wenn zusätzlich mittels Kombination von Magnetfeldsensoren und Beschleunigungssensoren die Orientierung des Objekts im Raum ermittelt wird und mit der durch die drei Drehratensensoren ermittelten Orientierung verglichen wird. Da das Magnetfeld an einem bestimmten Ort auf der Erde richtungs- und betragsmäßig bekannt ist, kann durch Magnetfeldsensoren, vorzugsweise wenn die die Sensoren tragende Vorrichtung zur Ruhe gebracht wurde, die Orientierung der Vorrichtung im Raum sehr genau bestimmt werden. Es ist dann möglich, die hieraus gewonnene Orientierung mit derjenigen aus den Signalen der Drehratensensoren ermittelten Orientierung zu vergleichen und letztere entsprechend zu korrigieren.

In einer ganz besonders vorteilhaften Weiterbildung dieses Erfindungsgedankens werden die durch die Magnetfeldsensoren erhaltenen Signale auf eine mögliche Fehlerbehaftung untersucht, bevor sie bei der Ausführung der Objektverfolgung Verwendung finden. Auf diese Weise wird verhindert, dass ein durch eine Außeneinwirkung, etwa einen Stromstoß oder dergleichen, verursachtes fehlerhaftes Signal der Magnetfeldsensoren bei der Ausführung der Objektverfolgung Berücksichtigung findet. Die Signale der Magnetfeldsensoren können in vorteilhafter Weise im Hinblick auf ihren absoluten Wert hin untersucht werden.

Wenn der absolute Wert der gemessenen Magnetfeldstärke den bekannten Wert des Erdmagnetfelds übersteigt, so kann hieraus geschlossen werden, dass diese Sensorsignale einen insbesondere durch Außeneinwirkung, etwa durch Annäherung eines Magneten, auftretenden Fehler enthalten und nicht berücksichtigt werden sollten. Des weiteren ist es denkbar und vorteilhaft, dass die Signale auf ihr Größenverhältnis und/oder die Orientierung zueinander untersucht werden. Auch anhand dessen lassen sich fehlerauslösende Einwirkungen detektieren, so dass die betreffenden Signale nicht berücksichtigt werden.

In besonders vorteilhafter Weise wäre es möglich, die Signale der Magnetfeldsensoren mit den Signalen von zusätzlich vorgesehenen Inertialsensoren zu vergleichen. Wenn, wie vorausgehend angedeutet, mittels Inertialsensoren die Erdbeschleunigung ermittelt wird, so ist es möglich, die Orientierung dieser Erdbeschleunigung mit der ebenfalls bekannten Orientierung der Magnetfeldstärke des Erdmagnetfelds zu vergleichen. Anhand dieses Vergleichs kann festgestellt werden, ob eine Störung vorliegt oder die aus den Werten ermittelte Orientierung des Objekts eine sehr genaue Orientierung darstellt.

Wenn die vorstehend erwähnte Untersuchung der Güte der Signale der Magnetfeldsensoren zu einem positiven Ergebnis fuhrt und diese Signale, insbesondere zusätzlich die Signale der Inertialsensoren, als Stützinformation für die Ermittlung der tatsächlichen Orientierung des Objekts im Raum verwendet werden, so werden sie mit der aus den drei Drehratensensoren ermittelten Orientierung verglichen. Dieser Vergleich kann in vorteilhafter Weise unter Ausführung eines Kalman-Filter-Algorithmus durchgeführt werden.

Hierbei erweist es sich als vorteilhaft, wenn die Messdaten sowohl der Inertialsensoren als auch der Magnetfeldsensoren während einer Ruhephase des Objekts genommen werden.

Gegenstand der Erfindung ist, wie eingangs erwähnt, auch eine Vorrichtung zur Ausführung des erfindungsgemäßen Verfahrens mit den Merkmalen der Ansprüche 11 bis 15.

Es wurde vorausgehend bereits darauf hingewiesen, dass das erfindungsgemäße Verfahren beziehungsweise eine erfindungsgemäße Vorrichtung beispielsweise bei Scannern Verwendung finden kann. Als besonders vorteilhaft erweist sich eine Verwendung im Bereich der Medizintechnik; es kann hiermit die Orientierung eines Ultraschallscannkopfs ermittelt werden, so dass beispielsweise ein sehr preiswerter zweidimensionaler Linienscanner verwendet werden kann, wobei der Scannkopf während der Ausführung des Scannvorgangs verschwenkt wird. Dieses Verschwenken kann dann über das erfindungsgemäße Verfahren festgestellt und ausgewertet werden. Auf diese Weise lässt sich ein sehr kostengünstiges Gerät darstellen, das gleichwohl dreidimensionale Informationen liefern kann.

In der Produktionstechnik existiert beispielsweise die Problematik manuell geführte Geräte entweder zu navigieren oder jedenfalls den Ablauf eines Montage- oder Fertigungsprozesses zu überwachen. So wäre die Anwendung zur Schrauberpositionserfassung denkbar, um festzustellen oder zu Überwachen, ob an einer bestimmten Stelle eine bestimmte Anzahl von Umdrehungen ausgeführt wurde. Zum Beispiel könnte die Erfindung auch im Zusammenhang mit einer sogenannten Erdrakete, bei der es sich um ein selbstlaufendes Bohrsystem für Erdbohrungen handelt, verwendet werden. Solchenfalls erweist es sich als vorteilhaft, wenn zusätzlich zu den Drehratensensoren Beschleunigungsensoren vorgesehen sind, welche eine translatorische Bewegung der Erdrakete ermitteln. Somit kann jederzeit die Position und die Orientierung einer herkömmlichen Erdrakete während eines automatisch ablaufenden Bohrvorgangs ermittelt und zu weiteren Steuerungsprozessen verwendet werden.

Eine besonders vorteilhafte Anwendung für das erfindungsgemäße Verfahren und eine erfindungsgemäße Vorrichtung stellen sogenannte Bedienpulte beispielsweise für Roboter oder Modellflugzeuge oder -autos dar. Solche Bedienpulte werden vom Benutzer beispielsweise am Bauch abgestützt getragen. Wenn die Orientierung des Bedienpults kontinuierlich ermittelt wird, so lässt sich beispielsweise eine Drehung des Benutzers um 90° detektieren und zu Steuerungsprozessen verwenden. Beispielsweise ist es dann möglich, dass sich Steuerungsvorgänge, etwa eine Hebelbewegung am Bedienpult, welche den Roboter vom Benutzer wegbewegt, leicht erlernt und unabhängig von der Orientierung des Benutzers stets beibehalten werden kann. Das "links/rechts-Umdenken", das jedermann von der Fernsteuerung eines Spielzeugautos her bekannt ist, könnte dann entfallen.

Des Weiteren konnte die Erfindung Verwendung in Form von miniaturisierten Orientierungserfassungssystemen auf dem Gebiet der medizinischen und orthopädischen Analyse finden.

Beispielsweise könnten am Bein eines Benutzers an verschiedenen Stellen miniaturisierte Sensoranordnungen vorgesehen werden. Damit könnte eine Analyse des Bewegungsablaufs beim Benutzer durchgeführt werden und insbesondere Bewegungen der Körperteile zueinander detektiert werden.

Weitere Merkmale, Einzelheiten und Vorteile der Erfindung ergeben sich aus den Patentansprüchen und der zeichnerischen Darstellung und nachfolgenden Beschreibung der Erfindung. In der Zeichnung zeigt:

Figur 1    die Darstellung der Rotation eines Vektors mittels Quaternionen,

Figur 2    ein Flussdiagramm, welches die Anwendung des Quaternionenalgorithmus verdeutlicht und

Figur 3    ein Flussdiagramm, welches die Ausführung des erfindungsgemäßen Verfahrens verdeutlicht.

Die Figuren 1 und 2 wurden bereits vorausgehend erläutert. Figur 3 zeigt ein Flussdiagramm für die Ausführung des erfindungsgemäßen Verfahrens.

Wie bereits erwähnt, wird zunächst das zu verfolgende Objekt, welches eine Vorrichtung mit drei Drehratensensoren trägt, im Raum zur Ruhe gebracht und derart mit einem ortsfesten Koordinatensystem referenziert, dass die aus den Signalen der drei Drehratensensoren ermittelten Winkel zunächst zu 0 gesetzt werden. Es wird dann während einer absoluten Ruhephase des Objekts und der das Objekt tragenden Sensoren ein Offset-Wert ermittelt, der bei jeder Abtastung, d. h. bei jeder Messdatennahme und Ermittlung von Drehwinkeln berücksichtigt wird. Es handelt sich hierbei um einen Driftvektor, dessen Komponenten die ermittelten Offset-Werte der Drehratensensoren umfassen.

Als ganz besonders vorteilhaft erweist es sich, dass erfindungsgemäß jedes mal, wenn ein Ruhen des Objekts detektiert und angenommen wird, die Offset-Werte der drei Drehratensensoren von neuem ermittelt und der weiteren Berechnung der Orientierung zugrundegelegt werden. Auf diese Weise lässt sich eine erhebliche Steigerung der Genauigkeit erreichen.

Ferner wird vor der Ausführung der Objektverfolgung die erwähnte Ausgleichsmatrix ermittelt, die einer Abweichung der drei Achsen der Drehratensensoren von einer angenommenen Orientierung zueinander und zu dem Objekt entspricht bzw. diese Abweichung genau ausgleichen soll.

Bei der Ausführung der Objektverfolgung werden also zu aufeinanderfolgenden Zeitpunkten, etwa mit einer Abtastrate von 10 bis 30 Hz, insbesondere 20 Hz, die Sensorsignale der drei Drehratensensoren bei denen es sich um Winkelgeschwindigkeiten handelt, durch einfache Integration in infinitesimale Drehwinkel umgerechnet. Dabei wird aber die Ausgleichsmatrix sowie die Ermittlung des jeweiligen Offset-Werts der jeweiligen Sensoren berücksichtigt, um zu einer erhöhten Genauigkeit bei der Ermittlung der Orientierung zu gelangen.

Anhand der infinitesimal kleinen Winkeländerungen, die jeweils aus den Messsignalen der drei Drehratensensoren ermittelt wurden, könnte nun nach dem Eulerschen Verfahren durch Angabe von drei Winkeln die Orientierung des verdrehten Koordinatensystems des Objekts zum Referenzkoordinatensystem ermittelt werden. Stattdessen erweist es sich als vorteilhaft, wenn zur Ermittlung der Orientierung ein Quaternionen-Algorithmus der eingangs beschriebenen Art durchgeführt wird. Hierdurch kann anstelle von drei nacheinander auszuführenden Drehungen eine einzige Transformation angenommen werden, was die Genauigkeit der hieraus gewonnenen Orientierung des Objektsystems weiter erhöht.

Im Ergebnis der Durchführung des Quaternionen-Algorithmus ist die Orientierung des Objekts im Raum gegeben.

Diese Orientierung ergibt bei einer Abtastfrequenz von 15 - 25 Hz, insb. bei etwa 20 Hz nach 5 min. der Objektverfolgung eine Genauigkeit, die besser ist als 4° Abweichung. Diese Genauigkeit ist für viele Anwendungen ausreichend.

Wie in Figur 3 angedeutet, ist es jedoch möglich, durch weitere Sensoren, etwa eine dreidimensionale Magnetfeldsensorik, zu beliebigen Zeitpunkten das auf das Objekt einwirkende Magnetfeld zu bestimmen, wobei es sich hierbei um das an einem Ort bekannte Erdmagnetfeld handelt. Des weiteren ist es denkbar, zusätzlich eine dreidimen-sionale Beschleunigungssensorik zur Messung der Erdbeschleunigung vorzusehen. Die Messsignale der Magnetfeldsensoren und der Beschleunigungssensoren lassen sich zu einem elektronischen dreidimensionalen Kompass kombinieren, der mit hoher Genauigkeit die Orientierung des Objekts im Raum angeben kann, wenn keine Störeffekte vorhanden sind, vorzugsweise wenn die Messwerte während eines Ruhens des Objekts genommen wurden. Die hieraus gewonnene Orientierung des Objekts im Raum lässt sich als Stützinformation für diejenige Orientierung, die nur durch die Signale der drei Drehratensensoren gewonnen wurde, verwenden. Zunächst werden die Messsignale der Magnetfeldsensoren und der Beschleunigungssensoren daraufhin untersucht, ob sie von Störungen beeinflusst sind oder nicht. Wenn dies nicht der Fall ist, so werden sie als bei der Ausführung des Verfahrens zu berücksichtigende Stützinformation mit der Orientierungsinformation, die aus den drei Drehratensensoren gewonnen wurde, verglichen. Hierfür wird in vorteilhafter Weise ein Kalman-Filter-Algorithmus verwendet. Hierbei handelt es sich um einen Schätzalgorithmus, bei dem die aus dem vorausgehend erwähnten dreidimensionalen Kompass ermittelten Informationen über die Orientierung des Objekts als korrekte Stützinformation zugrundegelegt werden, wenn sie mit denjenigen Informationen über die Orientierung verglichen werden, die aus den Drehratensensoren gewonnen wurden. Dies führt gerade bei Ausführung der Objektverfolgung über längere Zeiträume von vielen Minuten, insbesondere von mehr als 4 oder 5 min, zu einer Erhöhung der Genauigkeit.

Des weiteren ist in Figur 3 angedeutet, dass die Beschleunigungssensoren und deren Signale auch dazu verwendet werden können, durch zweifache Integration Informationen über eine translatorische Bewegung des Objekts im Raum zu ermitteln.

**Patentansprüche**

1.   Verfahren zum Ausführen einer Objektverfolgung, wobei mittels einer drei Drehratensensoren umfassenden Vorrichtung Messdaten ermittelt und hieraus durch Integration drei Drehwinkel zur weiteren Bestimmung der Orientierung des Objekts im Raum errechnet werden, wobei die Ermittlung der Messdaten und die Errechnung der drei Drehwinkel quasikontinuierlich entsprechend einer Abtastrate durchgeführt wird und wobei die Orientierung des Objekts im Raum angezeigt oder in an sich beliebiger Weise weiterer Datenverarbeitung zugeführt oder zu Steuerungsprozessen verwendet werden kann, **dadurch gekennzeichnet, dass** vor Beginn der Objektverfolgung und dann immer wieder sobald ein Ruhen der Vorrichtung für eine bestimmte Zeitdauer festgestellt wird ein Offset-Wert des Ausgangssignals der Drehratensensoren ermittelt wird und nachfolgend bis zur nächstfolgenden Ermittlung dieser Offset-Wert für die jeweiligen Drehratensensoren in Abzug gebracht wird, so dass er nicht in die Integration eingeht, und dass eine Abweichung der Orientierung der Achsen der drei Drehratensensoren der Vorrichtung von einer angenommenen Orientierung der Achsen zueinander und zu der Vorrichtung und der hieraus bei der Berechnung der Drehwinkel aus den Drehratensensorsignalen resultierende Fehler durch Anwendung einer diese Abweichung berücksichtigenden und vor Beginn der Objektverfolgung ermittelten Ausgleichsmatrix kompensiert wird.

2.   Verfahren nach Anspruch 1, **dadurch gekennzeichnet, dass** bei jeder Messdatennahme und Ermittlung der drei Drehwinkel ein Quaternionen-Algorithmus auf die drei Drehwinkel angewandt wird, um hieraus die aktuelle Orientierung des Objekts im Raum zu errechnen.

3. Verfahren nach Anspruch 1 oder 2, **dadurch gekennzeichnet, dass** eine Genauigkeit erreicht wird, die nach einer Zeitspanne von 5 min besser als 4° Abweichung ist.

4. Verfahren nach einem der vorstehenden Ansprüche, **dadurch gekennzeichnet, dass** zusätzlich mittels Inertialsensoren die Erdbeschleunigung gemessen wird.

5. Verfahren nach einem der vorstehenden Ansprüche, **dadurch gekennzeichnet, dass** zusätzlich mittels Magnetfeldsensoren die Orientierung des Objekts im Raum ermittelt wird und mit der durch die drei Drehratensensoren ermittelten Orientierung verglichen wird.

6. Verfahren nach Anspruch 5, **dadurch gekennzeichnet, dass** die durch die Magnetfeldsensoren erhaltenen Signale auf eine mögliche Fehlerbehaftung untersucht werden, bevor sie bei der Ausführung der Objektverfolgung Verwendung finden.

7. Verfahren nach Anspruch 6, **dadurch gekennzeichnet, dass** die Signale der Magnetfeldsensoren auf ihren absoluten Wert hin untersucht werden.

8. Verfahren nach Anspruch 6 oder 7, **dadurch gekennzeichnet, dass** die Signale auf ihr Größenverhältnis und/oder die Orientierung zueinander untersucht werden.

9. Verfahren nach Anspruch 6, 7 oder 8, **dadurch gekennzeichnet, dass** die Signale mit den Signalen der Intertialsensoren, welche die Erdbeschleunigung messen, verglichen werden.

10. Verfahren nach einem der Ansprüche 6 - 9, **dadurch gekennzeichnet, dass** die aus den Signalen der Magnetfeldsensoren und/oder der Intertialsensoren für die Messung der Erdbeschleunigung ermittele Orientierung über einen Kalman-Filter-Algorithmus verglichen werden.

11. Vorrichtung zur Ausführung des Verfahrens nach einem oder mehreren der vorstehenden Ansprüche, mit drei Drehratensensoren und mit Rechenmitteln zur Bestimmung der Orientierung im Raum, welche Integrationsmittel zur Integration der aus den Signalen der Drehratensensoren erhaltenen Messdaten umfassen, und mit Speichermitteln zum Speichern der Ausgleichsmatrix und der ermittelten Offset-Werte für die Drehratensensoren.

12. Vorrichtung nach Anspruch 11, **dadurch gekennzeichnet, dass** zusätzlich drei Beschleunigungssensoren vorgesehen sind, deren Signale zur Berechnung einer translatorischen Bewegung und/oder zur Ermittlung von zusätzlichen Informationen über die Orientierung des Objekts im Raum verwendbar sind.

13. Vorrichtung nach Anspruch 11 oder 12, **dadurch gekennzeichnet, dass** zusätzlich drei Magnetfeldsensoren vorgesehen sind, deren Signale zur Ermittlung von zusätzlichen Informationen über die Orientierung des Objekts im Raum verwendbar sind.

14. Vorrichtung nach einem der Ansprüche 11-13, **dadurch gekennzeichnet, dass** die Rechenmittel Mittel zur Ausführung eines Quaternionen-Algorithmus aufweisen.

15. Vorrichtung nach einem der Ansprüche 11-14, **dadurch gekennzeichnet, dass** die Rechenmittel Mittel zur Ausführung eines Kalman-Filter-Algorithmus aufweisen.

*Fig 1*

*Fig 2*

Fig 3